# FASCICULE DE BREVET EUROPEEN

(11) **EP 1 753 879 B1**
(45) Date de publication et mention de la délivrance du brevet: **15.07.2009**
(21) Numéro de dépôt: 05773252.1
(22) Date de dépôt: 25.05.2005
(51) Int. Cl.: C12Q 1/68

(54) **PROCEDE DE SELECTION OU D'OBTENTION DE PLANTES RESISTANTES AU PVMV**
VERFAHREN ZUR AUSWAHL BZW. GEWINNUNG VON PVMV-RESISTENTEN PFLANZEN
METHOD FOR SELECTING OR OBTAINING PLANTS WHICH ARE RESISTANT TO PVMV

(30) Priorité: 25.05.2004 FR 0405616
(43) Date de publication de la demande: 21.02.2007
(73) Titulaire: Genoplante-Valor, 91034 Evry Cédex (FR)
(72) Inventeur: CARANTA, Carole, F-84510 CAUMONT-SUR-DURANCE (FR); RUFFEL, Sandrine, F-34980 SAINT CLEMENT DE RIVIERE (FR); PALLOIX, Alain, 84250 Le Thor (FR); FABRE, Nicolas, F-13100 AIX-EN-PROVENCE (FR)
(74) Mandataire: Vialle-Presles, Marie José
(86) Numéro de dépôt international: PCT/FR2005/001296
(87) Numéro de publication internationale: WO 2005/118850

(56) Documents cités:
- WO-A-03/066900
- WO-A-20/04057941
- DUPRAT ANNE ET AL: "The Arabidopsis eukaryotic initiation factor (iso)4E is dispensable for plant growth but required for susceptibility to potyviruses." PLANT JOURNAL, vol. 32, no. 6, décembre 2002 (2002-12), pages 927-934, XP002309240 ISSN: 0960-7412
- LEONARD SIMON ET AL: "Complex formation between potyvirus VPg and translation eukaryotic initiation factor 4E correlates with virus infectivity" JOURNAL OF VIROLOGY, THE AMERICAN SOCIETY FOR MICROBIOLOGY, US, vol. 74, no. 17, septembre 2000 (2000-09), pages 7730-7737, XP002176524 ISSN: 0022-538X
- LELLIS A D ET AL: "Loss-of-susceptibility mutants of Arabidopsis thaliana reveal an essential role for eIF(iso)4E during Potyvirus infection" CURRENT BIOLOGY, CURRENT SCIENCE,, GB, vol. 12, no. 12, 25 juin 2002 (2002-06-25), pages 1046-1051, XP002217535 ISSN: 0960-9822
- RUFFEL S ET AL: "A natural recessive resistance gene against potato virus Y in pepper corresponds to the eukaryotic initiation factor 4E (eIF4E)" PLANT JOURNAL, BLACKWELL SCIENTIFIC PUBLICATIONS, OXFORD, GB, vol. 32, no. 6, décembre 2002 (2002-12), pages 1067-1075, XP002242998 ISSN: 0960-7412
- RUFFEL SANDRINE ET AL: "Structural analysis of the eukaryotic initiation factor 4E gene controlling potyvirus resistance in pepper: exploitation of a BAC library" GENE (AMSTERDAM), vol. 338, no. 2, 1 septembre 2004 (2004-09-01), pages 209-216, XP004527459 ISSN: 0378-1119

### SEQUENCE LISTING

<110> GENOPLANTE-VALOR
   CARANTA , Carole
   RUFFEL , Sandrine
   PALLOIX, Alain
   FABRE , Nicolas
<120> PROCEDE DE SELECTION OU D'OBTENTION DE PLANTES RESISTANTES AU PVMV
<130> MJPbv1516/17
<160> 13
<170> PatentIn version 3.1
<210> 1
   <211> 875
   <212> DNA
   <213> Capsicum annuum
<220>
   <221> misc_feature
   <222> (1) .. (815)
   <223> génotype Yolo Wonder ADNc du gène eIF4E (pvr2+)
<400> 1
   aaagtaaaaa aagcacacag caccaacaat ggcaacagct gaaatggaga aaacgacgac      60
   gtttgatgaa gctgagaagg tgaaattgaa tgctaatgag gcagatgatg aagttgaaga     120
   aggtgaaatt gttgaagaaa ctgatgatac gacgtcgtat ttgagcaaag aaatagcaac     180
   aaagcatcca ttagagcatt catggacttt ctggtttgat aatccagtgg cgaaatcgaa     240
   acaagctgct tggggtagct cgcttcgcaa cgtctacact ttctccactg ttgaagattt     300
   ttggggtgct tacaataata tccaccaccc aagcaagtta gttgtgggag cagacttaca     360
   ttgtttcaag cataaaattg agccaaagtg ggaagatcct gtatgtgcca atggagggac     420
   atggaaaatg agtttttcaa agggtaaatc tgataccagc tggctatata cgctgcttgc     480
   aatgattgga catcaattcg atcatgaaga tgaaatttgt ggagcagtag ttagtgtcag     540
   aggtaaggga gaaaaaatat ctttgtggac caagaatgct gcaaatgaaa cggctcaggt     600
   tagcattggt aagcaatgga agcagtttct ggattacagc gacagtgttg gcttcatatt     660
   tcacgacgat gcaaagaggc tcgacagaaa tgcaaagaat cgttacacag tataattctt     720
   gatgcaatgt cggaatataa gaaacacaat tcgtactgaa aagttgtagg cactagttta     780
   gtttctcata cgataaagct tctggtttga gtaccttgtg tattggtgtt tgcactttct     840
   aatcgcgtaa aattgtccgg ttttggattc aaaaa                                875
<210> 2
   <211> 228
   <212> PRT
   <213> Capsicum annuum
<220>
   <221> misc_feature
   <222> (1) .. (228)
   <223> génotype Yolo Wonder protéine eIF4E
<400> 2
   Met Ala Thr Ala Glu Met Glu Lys Thr Thr Thr Phe Asp Glu Ala Glu
   1               5                   10                  15
   Lys Val Lys Leu Asn Ala Asn Glu Ala Asp Asp Glu Val Glu Glu Gly
               20                  25                  30
   Glu Ile Val Glu Glu Thr Asp Asp Thr Thr Ser Tyr Leu Ser Lys Glu
           35                  40                  45
   Ile Ala Thr Lys His Pro Leu Glu His Ser Trp Thr Phe Trp Phe Asp
       50                  55                  60
   Asn Pro Val Ala Lys Ser Lys Gln Ala Ala Trp Gly Ser Ser Leu Arg
   65                  70                  75                  80
   Asn Val Tyr Thr Phe Ser Thr Val Glu Asp Phe Trp Gly Ala Tyr Asn
                   85                  90                  95
   Asn Ile His His Pro Ser Lys Leu Val Val Gly Ala Asp Leu His Cys
               100                 105                 110
   Phe Lys His Lys Ile Glu Pro Lys Trp Glu Asp Pro Val Cys Ala Asn
           115                 120                 125
   Gly Gly Thr Trp Lys Met Ser Phe Ser Lys Gly Lys Ser Asp Thr Ser
       130                 135                 140
   Trp Leu Tyr Thr Leu Leu Ala Met Ile Gly His Gln Phe Asp His Glu
   145                 150                 155                 160
   Asp Glu Ile Cys Gly Ala Val Val Ser Val Arg Gly Lys Gly Glu Lys
                   165                 170                 175
   Ile Ser Leu Trp Thr Lys Asn Ala Ala Asn Glu Thr Ala Gln Val Ser
               180                 185                 190
   Ile Gly Lys Gln Trp Lys Gln Phe Leu Asp Tyr Ser Asp Ser Val Gly
           195                 200                 205
   Phe Ile Phe His Asp Asp Ala Lys Arg Leu Asp Arg Asn Ala Lys Asn
       210                 215                 220
   Arg Tyr Thr Val
   225
<210> 3
   <211> 660
   <212> DNA
   <213> Capsicum annuum
<220>
   <221> misc_feature
   <222> (1) .. (660)
   <223> génotype Yolo Wonder ADNc du gène eIF(iso)4E (pvr6+)
<400> 3
   aaaacaatgg ccaccgaagc accaccaccg gtagacacga cggaggttcc gccgttcacg     60
   gcggcggaaa cggcggtgaa acagccacat aagctagaga gaaagtggac gttctggttc    120
   gataatcaat caaagccgaa acaaggcgcc gcttggggaa gttctcttaa aaaagcatat    180
   actttcgata ccgttgaaga attctggagt ttatatgatc agatattcaa gcccagcaag    240
   ttgactgtta atgcggattt tcatttgttc aaagctggga ttgagcccaa atgggaagat    300
   cctgagtgcg ctaatggtgg caagtggact gttacgagca gcagaaaagc taatcttgag    360
   actatgtggc ttgagactct gatggcattg gttggtgagc aatttgatga ttcagaagat    420
   atatgtggag tggttgctag cgtgcgtagg agtcaggata aactttcctt gtggactaag    480
   actgctacca atgaagcagc tcagatgggc attggtagga agtggaagga gatcattgat    540
   accgaaaaga tatcctatag tttccatgat gattctaaaa gggagaggtc agctaagagc    600
   cgatacaccg tgtgaatttc tttatcgtgt gggattgacc cctccccaag ttccagcaat    660
<210> 4
   <211> 202
   <212> PRT
   <213> Capsicum annuum
<220>
   <221> misc_feature
   <222> (1) .. (202)
   <223> génotype Yolo Wonder protéine eIF(iso)4E
<400> 4
   Met Ala Thr Glu Ala Pro Pro Pro Val Asp Thr Thr Glu Val Pro Pro
   1               5                   10                  15
   Phe Thr Ala Ala Glu Thr Ala Val Lys Gln Pro His Lys Leu Glu Arg
               20                  25                  30
   Lys Trp Thr Phe Trp Phe Asp Asn Gln Ser Lys Pro Lys Gln Gly Ala
           35                  40                  45
   Ala Trp Gly Ser Ser Leu Lys Lys Ala Tyr Thr Phe Asp Thr Val Glu
       50                  55                  60
   Glu Phe Trp Ser Leu Tyr Asp Gln Ile Phe Lys Pro Ser Lys Leu Thr
   65                  70                  75                  80
   Val Asn Ala Asp Phe His Leu Phe Lys Ala Gly Ile Glu Pro Lys Trp
                   85                  90                  95
   Glu Asp Pro Glu Cys Ala Asn Gly Gly Lys Trp Thr Val Thr Ser Ser
               100                 105                 110
   Arg Lys Ala Asn Leu Glu Thr Met Trp Leu Glu Thr Leu Met Ala Leu
           115                 120                 125
   Val Gly Glu Gln Phe Asp Asp Ser Glu Asp Ile Cys Gly Val Val Ala
       130                 135                 140
   Ser Val Arg Arg Ser Gln Asp Lys Leu Ser Leu Trp Thr Lys Thr Ala
   145                 150                 155                 160
   Thr Asn Glu Ala Ala Gln Met Gly Ile Gly Arg Lys Trp Lys Glu Ile
                   165                 170                 175
   Ile Asp Thr Glu Lys Ile Ser Tyr Ser Phe His Asp Asp Ser Lys Arg
               180                 185                 190
   Glu Arg Ser Ala Lys Ser Arg Tyr Thr Val
           195                 200
<210> 5
   <211> 23
   <212> DNA
   <213> Artificial sequence
<220>
   <223> amorce PCR 3'RACE de eIF(iso)4E de Capsicum annuum (génotype Yolo Wonder)
<400> 5
   aagtggactg ttacgagcag cag 23
<210> 6
   <211> 21
   <212> DNA
   <213> Artificial sequence
<220>
   <223> amorce 3' de eIF(iso)4E de Capsicum annuum (génotype Yolo Wonder)
<400> 6
   attgctggaa cttggggagg g 21
<210> 7
   <211> 21
   <212> DNA
   <213> Artificial sequence
<220>
   <223> amorce 5' de eIF(iso)4E de Capsicum annuum (génotype Yolo Wonder)
<400> 7
   aaaacaatgg ccaccgaagc a 21
<210> 8
   <211> 25
   <212> DNA
   <213> Artificial sequence
<220>
   <223> amorce PCR sens de eIF(iso)4E de Capsicum annuum
<400> 8
   atggccaccg aagcaccacc accgg 25
<210> 9
   <211> 25
   <212> DNA
   <213> Artificial sequence
<220>
   <223> amorce PCR anti-sens de eIF(iso)4E de Capsicum annuum
<400> 9
   tcacacggtg tatcggctct tagct 25
<210> 10
   <211> 21
   <212> DNA
   <213> Artificial sequence
<220>
   <223> amorce RT-PCR sens
<400> 10
   ccgatctcaa gccactctcc g 21
<210> 11
   <211> 22
   <212> DNA
   <213> Artificial sequence
<220>
   <223> amorce RT PCR anti-sens
<400> 11
   cctgaagctg tggcaggagt tg 22
<210> 12
   <211> 17
   <212> DNA
   <213> Artificial sequence
<220>
   <223> amorce RT PCR sens dégénérée
<220>
   <221> misc_feature
   <222> (3) .. (3)
   <223> n = inosine
<220>
   <221> misc_feature
   <222> (6) .. (6)
   <223> r = g ou a
<220>
   <221> misc_feature
   <222> (9) .. (9)
   <223> n = a ou g ou c ou t, inconnu, ou autre
<220>
   <221> misc_feature
   <222> (12) .. (12)
   <223> n = a ou g ou c ou t, inconnu, ou autre
<220>
   <221> misc_feature
   <222> (15) .. (15)
   <223> y = t ou c
<400> 12
   ggnaargcnc cntayat 17
<210> 13
   <211> 20
   <212> DNA
   <213> Artificial sequence
<220>
   <223> amorce RT PCR anti-sens dégénérée
<400> 13
   cgcgctaatg acatatcggt 20

## Description

La présente invention concerne un procédé de sélection ou d'obtention de plantes résistantes à un virus du genre *Potyvirus* via la combinaison de mutations dans les gènes *eIF4E* et *eIF(iso)4E.*

Les potyvirus forment le groupe de phytovirus le plus important. Ils représentent environ 1/3 des virus végétaux et sont capables d'infecter plus de 30 familles de plantes (SHUKLA et al., 1994, In Genome structure, variation and function in the Potyviridae, Ed. CAB International, Wallingford, UK). La transmission des potyvirus est réalisée par les pucerons (par exemple, *Myzus persicae*) selon le mode non-persistant. L'infection provoque des anomalies de coloration des feuilles (mosaïques, jaunissement des nervures), des déformations des feuilles, des nécroses nervaires pouvant conduire à la nécrose de la plante entière, et donc à une réduction importante de la taille de la plante malade et une perte de productivité. Les fleurs, les graines et les fruits sont eux aussi affectés par de nombreux potyvirus, ce qui a pour conséquences une réduction de la fertilité, la transmission des potyvirus par la graine et des déformations, décolorations et nécroses des fruits.

Les potyvirus ont une structure filamenteuse non-enveloppée (LANGENBERG et ZHANG, J. Struct. Biol. 118(3) : 243-247, 1997) de 680 à 900 nm de long et de 11 à 15 nm de large (DOUGHERTY et CARRINGTON, Ann. Rev. Phytopathol. 26 : 123-143, 1988 ; RIECHMANN et al., J. Gen. Virol. 73 : 1-16, 1992). Le génome viral est constitué d'un ARN simple brin sens d'une longueur approximative de 10 kb. L'ARN simple brin possède à son extrémité 3' une queue poly A et se lie en 5' à une protéine virale appelée VPg pour Viral Protein genome-linked (MURPHY et al., Virol. 178 : 285-288, 1990 ; TAKAHASHI et al., Virus genes 14(3) : 235-243, 1997). L'ARN viral code pour une polyprotéine maturée en 10 protéines fonctionnelles impliquées dans les différentes étapes du cycle infectieux : clivage de la polyprotéine, réplication du génome viral, mouvement de cellule-à-cellule, mouvement longue distance, transmission par pucerons et inhibition du « RNA silencing », etc...

Les solanacées, cucurbitacées, fabacées, crucifères et composées sont particulièrement sensibles aux potyvirus. Par exemple, les solanacées et plus particulièrement la tomate et le piment (ou poivron) sont infectées par plusieurs potyvirus distincts : le virus Y de la pomme de terre (*Potato virus Y -* PVY) est présent sur l'ensemble des zones de culture alors que les autres sont cantonnés à un continent (*Tobacco etch virus* (TEV), Pepper mottle virus sur le continent américain, *Pepper veinal mottle virus* (PVMV) et *Potyvirus E* en Afrique, et *Chili veinal mottle virus* en Asie). Cette compartimentation n'est cependant plus absolue, plusieurs potyvirus ayant été identifiés hors de leur zone d'origine. Le PVMV a été tout d'abord isolé en 1971 sur piment et pétunia au Ghana. Depuis, il a été identifié dans plusieurs pays africains, principalement dans les régions sub-sahariennes, mais également en Tunisie et Ethiopie (BRUNT et al., Ann. Appl. Biol. 69 : 235-243, 1971 ; BRUNT et al., Ann. Appl. Biol. 88 : 115-119, 1978 ; GORSANE et al., Plant Mol. Biol. Reptr. 17 : 149-158, 1999). Ses hôtes naturels sont principalement des solanacées, dont le piment mais également la tomate, le tabac et l'aubergine.

A l'heure actuelle et contrairement aux infections fongiques ou bactériennes, il n'existe pas de moyen de lutte directe contre les virus. Les stratégies employées consistent à éliminer les vecteurs viraux tels que les pucerons, et/ou à sélectionner et à cultiver des plantes résistantes au(x) virus le(s) plus fréquent(s) dans la zone de culture.

Du fait de l'internationalisation du marché de la semence, il est de plus en plus indispensable pour les sélectionneurs de créer des variétés multi-résistantes. Plus généralement, considérant l'importance économique des infections par potyvirus et l'absence de moyens directs de lutte contre ce type d'infection, la recherche de variétés végétales multirésistantes constitue un des axes principaux de l'amélioration des plantes.

CARANTA et al. (Phytopathol. 86(7) : 739-743, 1996) ont montré chez le piment que la combinaison de certains loci permettait d'élargir le spectre de résistance en conférant des résistances à de nouveaux potyvirus. Ainsi, des plantes résistantes au PVMV ont été identifiées dans la descendance haploïde doublée issue du croisement entre les variétés de *Capsicum annuum* Perennial et Florida VR2, toutes deux sensibles au PVMV. Les analyses génétiques ont montré que la résistance au PVMV résultait de l'association du gène récessif *pvr2* (issu de Florida VR2 et contrôlant la résistance aux PVY pathotypes 0 et 1 et au TEV) avec un autre facteur récessif, nommé *pvr6,* issu de Perennial.

Face à une agression par un agent pathogène (virus, bactéries, champignons ou nématodes), la plante possède plusieurs stratégies pour se défendre ou résister à l'infection. On distingue la résistance non-hôte (lorsque toutes les entités d'une espèce sont résistantes à un pathogène donné) de la résistance hôte (lorsque au moins une entité de l'espèce est sensible à une souche de l'agent pathogène). Il est possible de distinguer deux modalités de mise en place de la résistance hôte selon que les gènes impliqués sont dominants ou récessifs.

Le "modèle gène-à-gène" décrit par FLOR (Ann. Rev. Phytopathol. 9 : 275-296, 1971) fait intervenir un gène majeur dominant de la plante. La résistance est conditionnée par la présence de ce gène et d'un gène d'avirulence spécifique correspondant, chez l'agent pathogène. Lorsque ces deux gènes sont simultanément présents, la résistance de la plante hôte est induite et est souvent associée à une réaction d'hypersensibilité (nécrose) localisée au site d'infection.

La résistance peut également être contrôlée par des gènes de plantes récessifs. Singulièrement, on estime que près de la moitié des résistances aux potyvirus sont récessives alors que pour les autres agents pathogènes, cette proportion n'atteint que 20% en moyenne. FRASER (Euphytica 63 : 175-185, 1992) a émis l'hypothèse, connue sous le nom de « modèle négatif de Fraser », que les résistances récessives résulteraient d'un déficit ou d'une altération spécifique du produit d'un gène de l'hôte nécessaire à l'accomplissement du cycle viral dans la plante.

La protéine VPg des potyvirus joue un rôle important dans les interactions potyvirus/plante-hôte, et notamment dans la virulence de certaines souches de potyvirus vis-à-vis de certaines plantes-hôtes. Il a été montré que des potyvirus portant des mutations dans la protéine VPg pouvaient contourner des gènes récessifs de résistance impliqués dans des mécanismes aussi variés que l'inhibition de la multiplication virale, l'inhibition de la migration des potyvirus de cellule à cellule ou l'inhibition de leur migration à longue distance. Ceci a été montré chez les couples TVMV/*Nicotiana tabacum* (gène *va*), PVY/tomate (gène *pot*-1), PVY/piment (gène *pvr2*), LMV/Laitue (gène mol) et PSbMV/pois (gène *sbm*1) (KELLER et al., Mol. Plant-Microbe Interact. 11 : 124-130 20, 1998; MOURY et al., Mol. Plant-Microbe Interact. 17(3) : 322-329, 2004; REDONDO et al., Mol. Plant-Microbe Interact. 14 : 804-810, 2001 ; NICOLAS et al., Virol. 237 : 452-459 35, 1997 ; MASUTA et al., Phytopathol. 89 : 118-123, 1999).

Parmi les facteurs de la plante hôte capables d'interagir avec la protéine VPg des potyvirus, figure notamment le facteur eucaryote d'initiation de la traduction des ARN 4E (eIF4E), et en particulier les isoformes appelées eIF4E et eIF(iso)4E (WITTMAN et al., Virol. 234 : 84-92, 1997 ; SCHAAD et al., Virol. 273 : 300-306, 2000 ; LELLIS et al., Curr. Biol. 12 : 1046-1051, 2002 ; DUPRAT et al., Plant J. 32 : 927-934, 2002). Chez les plantes, eIF4E appartient au complexe eIF4F (composé de eIF4E et eIF4G) qui permet de faire le lien entre la coiffe des ARN (m7GpppN) et la machinerie d'initiation de la traduction (BROWNING, Plant Mol. Biol. 32(1-2) : 107-143, 1996). Deux complexes eIF4F (nommés eIF4F et eIF(iso)4F) ont été purifiés à partir de cellules végétales et leurs sous-unités respectives ont été nommées eIF4E (sous-unité p26) et eIF(iso)4E (sous-unité p28), pour la sous-unité se liant à la coiffe des ARN, et eIF4G et eIF(iso)4G pour l'autre sous-unité. eIF4E et eIF(iso)4E, qui apparaissent impliqués dans la traduction de différents types d'ARN, appartiennent à une petite famille multigénique chez les plantes, comprenant 4 gènes chez *Arabidopsis thaliana,* dont trois codent pour eIF4E et une pour l'isoforme eIF(iso)4E (RODRIGUEZ et al., Plant J. 13(4) : 465-473, 1998).

Les protéines eIF4E et eIF(iso)4E possèdent toutes deux le domaine conservé dénommé IF4E. Ce domaine est référencé sur la base de données PFAM (BATEMAN et al., Nucleic Acids Res. 30 : 276-280, 2002) sous le numéro PFAM 01652. Cependant, les protéines eIF4E appartenant à la même sous-famille (i.e., 4E ou (iso)4E) et issues d'espèces différentes présentent un pourcentage d'identité (61 à 86% d'identité entre les protéines eIF4E, 59 à 82% d'identité entre les protéines eIF(iso)4E) supérieur à celui que présentent entre elles les protéines eIF4E et eIF(iso)4E issues de la même espèce (42 à 51% d'identité) (RUFFEL et al., Gene, in press, 2004).

Dans le cadre de l'exposé qui va suivre, on se référera, à titre de protéines représentatives respectivement de la sous-famille eIF4E, et de la sous-famille eIF(iso)4E, à la protéine eIF4E et à la protéine eIF(iso)4E de *Capsicum annuum* .

La séquence d'ADNc codant pour la protéine eIF4E de *Capsicum annuum* (GenBank n° AY122052) est représentée dans la liste de séquences en annexe sous le numéro SEQ ID NO : 1, et la séquence polypeptidique déduite est représentée sous le numéro SEQ ID NO : 2.

La séquence d'ADNc codant pour la protéine eIF(iso)4E de *Capsicum annuum* est représentée dans la liste de séquences en annexe sous le numéro SEQ ID NO:3, et la séquence polypeptidique déduite est représentée sous le numéro SEQ ID NO : 4.

On définit ici comme protéine eIF4E, toute protéine comprenant un domaine IF4E, et dont la séquence peptidique possède au moins 60 %, de préférence au moins 65%, de manière tout à fait préférée au moins 70% d'identité globale avec la protéine eIF4E de *Capsicum annuum* (SEQ ID NO : 2)

On définit comme protéine eIF(iso)4E toute protéine comprenant un domaine IF4E, et dont la séquence polypeptidique possède au moins 59 %, de préférence au moins 65 %, de manière tout à fait préférée au moins 70 % d'identité avec la protéine eIF(iso)4E de *Capsicum annuum* (SEQ ID NO : 4).

Les pourcentages d'identité auxquels il est fait référence ici sont des pourcentages d'identité globale, déterminés en utilisant le programme PILEUP de GCG (Genetics Computer Group) sur un alignement de séquences contenant la totalité de la séquence de référence SEQ ID NO : 2 ou SEQ ID NO : 4.

Il a été rapporté que des mutations altérant soit eIF4E soit eIF(iso)4E induisent une résistance récessive à certaines espèces de potyvirus. Par exemple, il a été montré que la disruption du gène codant pour l'isoforme eIF(iso)4E chez *Arabidopsis thaliana* induisait la résistance au *Turnip mosaic virus* (TuMV), au TEV et au *Lettuce mosaic virus* (LMV), mais n'avait pas d'effet sur la résistance au *Tomato block ring virus* (TBRV, genre *Népovirus*) ou au *Cucumber mosaic virus* (CMV, genre *Cucumovirus*) (DUPRAT et al., Plant J. 32 : 927-934, 2002 ; LELLIS et al., Curr. Biol. 12 : 1046-1051, 2002). NICAISE et al. (Plant Physiol., 132 : 1272-1282, 2003), ont montré que des plantes résistantes au virus de la mosaique de la laitue (LMV) portaient des mutations ponctuelles dans le gène *eIF4E* ; en revanche, la séquence du gène *eIF(iso)4E* chez les plants de laitue résistants ne différait pas de celle des plantes sensibles.

RUFFEL et al. (Plant J. 32 : 1067-1075, 2002) ainsi que la Demande PCT WO 03/066900 montrent que la résistance récessive au TEV et au PVY conférée par le gène récessif *pvr2* est associée à des variations de séquences dans *eIF4E,* et notamment dans une région de cette protéine qui est très conservée entre les protéines eIF4E issues de diverses espèces végétales.

Les Inventeurs ont maintenant mis en évidence chez le piment, la coségrégation entre le locus *pvr6* et le gène *eIF(iso)4E.* Ils ont constaté que les plantes présentant une résistance au PVMV possédaient, en plus de l'allèle muté codant pour le facteur de traduction eIF4E (allèle de résistance *pvr2*), tel que décrit dans la Demande de brevet WO 03/066900, une mutation dans la séquence codant pour eIF(iso)4E.

Cette mutation consiste en une délétion de 82 pb dans la séquence codante, incorporant un codon stop au 51^{ème} acide aminé sur les 202 de la protéine sauvage. Seuls les 29 premiers acides aminés sont conservés entre la protéine eIF(iso)4E sauvage et la protéine mutée, indiquant que la protéine mutée n'est plus fonctionnelle.

Le facteur récessif *pvr6,* précédemment décrit comme ne conférant pas isolément de résistance au PVMV, mais capable de conférer cette résistance lorsqu'il était associé au facteur récessif *pvr2* (CARANTA et *al*., 1996, précité), correspond donc au gène *eIF(iso)4E.* Cette correspondance entre *pvr6* et *eIF(iso)4E* est inattendue, dans la mesure où dans le cas des mutants de *eIF4E* ou de *eIF(iso)4E* identifiés jusqu'à présent, la mutation d'un seul de ces deux gènes suffisait à conférer une résistance récessive à un ou plusieurs potyvirus particuliers.

Il apparaît donc que certains potyvirus, dont le PVMV est le premier exemple, se comportent différemment vis-à-vis de l'utilisation d'eIF4E et d'eIF(iso)4E pour accomplir leur cycle infectieux, de ce qui avait été observé jusqu'à présent. Alors que les potyvirus étudiés jusqu'à présent montraient une spécialisation dans l'utilisation, dans une plante hôte, soit d'eIF4E soit d'eIF(iso)4E, le PVMV apparaît capable d'utiliser aussi bien eIF4E qu'eIF(iso)4E.

La présente invention concerne un procédé de sélection de plantes résistantes au PVMV, **caractérisé en ce qu**'il comprend la recherche, dans les plantes à tester, des formes des protéines eIF4E, et eIF(iso)4E présentes chez lesdites plantes, et la sélection des plantes qui :
a) n'expriment aucune protéine eIF4E (dénommée ci-après : « protéine eIF4E de type sauvage ») comprenant une région définie par la séquence générale (I) suivante : DX₁X₂X₃X₄KSX₅QX₆AWGSSX₇RX₈X₉YTFSX₁₀VEX₁₁FWX₁₂X₁₃YNNIHX₁₄PSKLX₁₅X₁₆ GAD
   dans laquelle :
   - X_{1,} X_{2,} X_{3,} X_{4,} X_{6,} X₇, X_{8,} X₉, X₁₀, X_{12,} X₁₃, X_{15,} et X₁₆ représentent chacun un acide aminé neutre ;
   - X₅ et X₁₄ représentent un acide aminé basique ;
   - X₁₁ représente un acide aminé acide ;
   - D, K, S, Q, A, W, G, R, Y, T, F, V, E, N, I, H, P, et
      L ont leur signification usuelle en code 1-lettre ; et expriment une protéine eIF4E mutante comprenant une région dérivée de celle définie par la séquence (I) ci-dessus, par substitution d'au moins un acide aminé neutre de ladite séquence (I) par un acide aminé chargé, de préférence un acide aminé basique et/ou substitution d'au moins un acide aminé chargé de ladite séquence (I) par un acide aminé neutre ou un acide aminé de charge opposée ;
      et
b) n'expriment aucune protéine eIF(iso)4E fonctionnelle.

On définit ici comme « acide aminé neutre », tout acide aminé choisi parmi les suivants : alanine, valine, leucine, isoleucine, proline, tryptophane, phénylalanine, méthionine, glycine, sérine, thréonine, tyrosine, cystéine, glutamine, asparagine. On définit comme « acide aminé chargé », tout acide aminé choisi parmi les suivants : histidine, lysine, arginine, glutamate, aspartate. Parmi ces acides aminés chargés, histidine, la lysine ou l'arginine sont des acides aminés basiques, et le glutamate et l'aspartate sont des acides aminés acides.

On définit ici comme « protéine eIF(iso)4E fonctionnelle » une protéine eIF(iso)4E capable de former un complexe avec eIF(iso4G), et de se lier à la coiffe des ARNs. Des plantes n'exprimant aucune protéine eIF(iso)4E fonctionnelle peuvent, soit n'exprimer aucune protéine eIF(iso)4E, soit exprimer uniquement une protéine eIF(iso)4E non-fonctionnelle.

L'absence d'expression d'une protéine eIF(iso)4E peut résulter par exemple de la délétion de la séquence codant pour cette protéine, ou de l'absence de transcription et/ou de traduction du gène *eIF(iso)4E.* La non-fonctionnalité d'une protéine eIF(iso)4E peut résulter par exemple d'une mutation dans la séquence codant pour cette protéine.

En ce qui concerne cette protéine eIF(iso)4E non-fonctionnelle, on sélectionne préférentiellement les plantes chez lesquelles ladite protéine eIF(iso)4E non-fonctionnelle est dépourvue d'une portion de la protéine eIF(iso)4E sauvage comprenant un ou plusieurs des 8 résidus tryptophanes du domaine IF4E conservés entre les protéines eIF(iso)4E eucaryotes. Ces résidus tryptophanes correspondent à ceux situés, dans la séquence SEQ ID NO : 4, aux positions 34, 37, 50, 67, 96, 107, 121, et 156. Ils peuvent être facilement localisés par l'homme du métier dans toute autre protéine eIF(iso)4E à partir d'un alignement de séquences.

Avantageusement, la portion délétée comprend une ou plusieurs des régions du domaine IF4E correspondant aux positions 31-36, 47-52, 64-69, 93-98, 118-123, et 153-158 de la séquence SEQ ID NO : 4.

De manière particulièrement avantageuse, la portion délétée comprend les résidus du domaine IF4E correspondant aux positions 30-202 de la séquence de référence SEQ ID NO : 4.

Cette délétion dans la protéine eIF(iso)4E peut résulter d'une délétion dans la portion codante du gène *eIF(iso)4E ;* elle peut également résulter d'une mutation ponctuelle dans le gène *eIF(iso)4E,* ou de l'insertion d'une séquence exogène, introduisant un codon stop prématuré, et/ou un décalage du cadre de lecture. Selon le type de mutation concernée, et la position de cette mutation, la protéine eIF(iso)4E non-fonctionnelle qui en résulte peut comprendre, à la place de la portion délétée, une séquence non-apparentée.

En ce qui concerne la protéine eIF4E, on sélectionne préférentiellement les plantes contenant une protéine eIF4E mutante comprenant une région dérivée de celle définie par la séquence (I) ci-dessus, par substitution :
- d'au moins un des acides aminés X_{1,} X_{2,} X₃ ou X_{4,} de ladite séquence (I) par un acide aminé chargé ; et
- d'au moins un des autres acides aminés neutres de ladite séquence (I) par un acide aminé chargé ; et/ou
- d'au moins un des acides aminés chargés de ladite séquence (I) par un acide aminé neutre ou un acide aminé de charge opposée.

De manière particulièrement préférée, on sélectionne les plantes contenant une protéine eIF4E mutante comprenant une région dérivée de celle définie par la séquence (I) ci-dessus, par substitution :
- de l'acide aminé neutre X₃ de la séquence (I) par un acide aminé basique ;
- de l'acide aminé neutre X₇ de la séquence (I) par un acide aminé basique ;
- du résidu aspartate en position C-terminale de la séquence (I) par un acide aminé neutre.

La détection de la forme des protéines eIF4E et eIF(iso)4E présente dans la plante à tester peut s'effectuer par exemple à l'aide d'anticorps spécifiquement dirigés soit contre la forme sauvage soit contre la forme mutante de la protéine eIF4E ou de la protéine eIF(iso)4E.

Cependant, il est généralement plus commode d'effectuer cette détection par recherche des formes alléliques des gènes *eIF4E* et *eIF(iso)4E* présentes chez la plante à tester.

La présente invention concerne un procédé de sélection de plantes résistantes au PVMV, **caractérisé en ce qu**'il comprend la recherche, dans les plantes à tester, des formes alléliques des gènes *eIF4E,* et *eIF(iso)4E* présentes ou exprimées chez lesdites plantes, et la sélection des plantes qui ne présentent ou n'expriment aucun allèle du gène eIF4E codant pour une protéine de eIF4E de type sauvage, telle que définie ci-dessus, et qui expriment un allèle du gène *eIF4E* codant pour une protéine eIF4E mutante, telle que définie ci-dessus, et qui en outre, ne présentent ou n'expriment aucun allèle du gène *eIF(iso)4E* codant pour une protéine eIF(iso)4E fonctionnelle, telle que définie ci-dessus, et, le cas échéant expriment un allèle du gène eIF(iso)4E codant pour une protéine eIF(iso)4E non-fonctionnelle, telle que définie ci-dessus.

La détection des formes alléliques des gènes *eIF4E* et *eIF(iso)4E* présentes chez la plante à tester peut s'effectuer par des méthodes classiques, connues en elles-mêmes de l'homme du métier. On peut par exemple utiliser des sondes polynucléotidiques capables de s'hybrider sélectivement soit avec l'allèle sauvage soit avec l'allèle mutant de *eIF4E* ou *eIF(iso)4E,* ou des amorces d'amplification permettant l'amplification de *eIF4E* ou de *eIF(iso)4E* ou de la portion de ces gènes contenant la mutation recherchée ; la présence ou l'absence de cette mutation peut ensuite être détectée sur le produit d'amplification, par exemple par séquençage de celui-ci,
ou, selon la nature de la mutation recherchée, par détermination de la taille du produit d'amplification, ou digestion avec une enzyme de restriction reconnaissant une séquence-cible présente exclusivement, soit dans l'allèle sauvage, soit dans l'allèle mutant.

La présente invention a également pour objet des procédés d'obtention de plantes résistantes au PVMV.

Selon une première variante d'un procédé conforme à l'invention, il comprend :
- le remplacement des allèles du gène *eIF4E* présents chez ladite plante par des allèles codant pour une protéine eIF4E mutante telle que définie ci-dessus ; et
- le remplacement des allèles du gène *eIF(iso)4E* présents chez ladite plante par des allèles codant pour une protéine eIF(iso)4E mutante non-fonctionnelle telle que définie ci-dessus, ou l'inactivation directe ou épigénétique dudit gène *eIF(iso)4E.*

Selon une seconde variante d'un procédé conforme à l'invention, il est mis en oeuvre à partir d'une plante dont tous les allèles du gène *eIF4E* codent pour une protéine eIF4E mutante telle que définie ci-dessus, et comprend le remplacement des allèles du gène *eIF(iso)4E* présents chez ladite plante par des allèles codant pour une protéine eIF(iso)4E mutante non-fonctionnelle telle que définie ci-dessus, ou l'inactivation directe ou épigénétique dudit gène *eIF(iso)4E.*

Selon une troisième variante d'un procédé conforme à l'invention, il est mis en oeuvre à partir d'une plante dont tous les allèles du gène *eIF(iso)4E* codent pour une protéine eIF(iso)4E mutante telle que définie ci-dessus, ou à partir d'une plante dans laquelle ledit gène *eIF(iso)4E* a été inactivé, et comprend le remplacement des allèles du gène *eIF4E* présents chez ladite plante par des allèles codant pour une protéine eIF4E mutante telle que définie ci-dessus.

Par « remplacement des allèles » du gène *eIF4E*
ou du gène *eIF(iso)4E* on entend ici toute modification génétique résultant en la substitution de la séquence codante initiale du gène concerné par la séquence mutante souhaitée, quelle que soit la méthode utilisée pour effectuer cette modification.

Par « inactivation » du gène *eIF(iso)4E* on entend ici toute modification génétique résultant en l'absence d'expression d'une protéine eIF(iso)4E. On définit cette inactivation comme « directe » si elle résulte d'une modification du gène *eIF(iso)4E* (par exemple au niveau de la séquence codante ou de la séquence promotrice dudit gène), et comme « épigénétique » si elle ne résulte pas d'une modification du gène *eIF(iso)4E.* L'inactivation épigénétique, également dénommée « extinction » peut résulter notamment d'un blocage de la transcription (extinction transcriptionnelle ou TGS pour :
« Transcriptional Gene Silencing »), ou de la dégradation spécifique des ARNm (extinction post-transcriptionnelle ou PTGS pour « Post-Transcriptional Gene Silencing »).

Le remplacement des allèles des gènes *eIF4E* ou *eIF(iso)4E* par les allèles mutants souhaités peut s'effectuer par des techniques connues en elles-mêmes, faisant appel à la transgenèse, telles que la recombinaison homologue, ou à la mutagenèse, telles que le TILLING (Targeting Induced Local Lesions IN Genomes ; McCALLUM et al., Plant Physiol. 123 : 439-442, 2000), ou dans le cas du gène *eIF(iso)4E,* la mutagenèse par insertion.

L'inactivation directe du gène *eIF(iso)4E* peut également s'effectuer par les mêmes techniques

L'extinction du gène *eIF(iso)4E* peut s'effectuer par suppression sens ou antisens, ou par l'utilisation de RNAi (NISHIKURA, Cell 107 : 415-418, 2001 ; TENLLADO et al., Virus Res. 102 : 85-96, 2004 ; ZAMORE, Methods Mol. Biol. 252 : 533-543, 2004).

La présente invention a également pour objet des plantes résistantes au PVMV, susceptibles d'être obtenues par un procédé conforme à l'invention.

Ceci inclut notamment :
- les plantes dans lesquelles les allèles des gènes *eIF4E* présents sont des allèles mutants tels que définis ci-dessus, et les allèles du gènes *eIF(iso)4E* présents sont des allèles mutants non-fonctionnels tels que définis ci-dessus, à l'exception des doubles mutants *pvr2 pvr6* décrits dans la publication de CARANTA et *al*. (1996, précité) ;
- les plantes dans lesquelles les allèles du gène *eIF4E* présents sont des allèles mutants, tels que définis ci-dessus, et les allèles du gène *eIF(iso)4E* ont été inactivés directement ;
- les plantes dans lesquelles les allèles du gène *eIF4E* présents sont des allèles mutants, tels que définis ci-dessus, et qui sont transformées par une séquence (sens, antisens, ou pouvant être transcrite en RNAi), capable d'éteindre l'expression du gène *eIF(iso)4E.*

Les procédés de sélection ou d'obtention de plantes résistantes au PVMV conformes à l'invention sont applicables à toutes les plantes que l'on souhaite protéger contre le PVMV, et plus particulièrement aux plantes de la famille des solanacées telles que le piment, la tomate ou l'aubergine, des cucurbitacées comme le melon, des crucifères, des fabacées telles que le pois, et des composées.

En outre, ils peuvent également être applicables pour la protection des plantes contre d'autres potyvirus, qui comme le PVMV peuvent utiliser indifféremment *eIF4E* ou *eIF(iso)4E* pour accomplir leur cycle infectieux, à titre d'exemple, on citera le potyvirus BCMV (Bean *common mosaic virus*), ou le potyvirus AzMV (*Azukini mosaic virus*) (FISHER et KYLE, Theor. Appl. Genet. 92 : 204-212, 1996).

La présente invention sera mieux comprise à l'aide du complément de description qui va suivre, qui se réfère à des exemples illustrant l'implication de *eIF4E* et *eIF(iso)4E* dans la résistance au PVMV, et la mise en oeuvre de procédés conformes à l'invention de sélection et d'obtention de plantes résistantes au PVMV.

### EXEMPLE 1 : OBTENTION DE L'ADNC DE eIF(ISO)4E (pvr6⁺) CHEZ LE PIMENT

L'ARN total issu du génotype Yolo Wonder de piment sensible aux potyvirus a été isolé à partir de 100 à 200 mg de feuilles en utilisant le réactif TRI-Reagent (Sigma-Aldrich, St Louis).

L'extrémité 3' de l'ADNc de eIF(iso)4E de piment a été obtenue en utilisant la technique PCR de 3'RACE (kit GIBCO/BRL Life technologies 3'RACE system, version 2.0) en utilisant une amorce 5'-AAGTGGACTGTTACGAGCAGCAG-3' (SEQ ID NO : 5) conçue à partir de l'alignement des séquences d'ADNc de eIF(iso)4E *d'Arabidopsis thaliana* (EMBL n°accession : Y10547), de *Lactuca sativa* (GenBank n°accession : AF530163) et de *Lycopersicon esculentum* (TIGR : TC126316).

L'ADNc pleine longueur du gène *eIF(iso)4E* de piment a été amplifié par RT-PCR avec une amorce conçue à partir de la séquence du produit 3'RACE (5'-ATTGCTGGAACTTGGGGAGGG-3', SEQ ID NO : 6) et une amorce conçue à partir de l'extrémité 5' UTR de la séquence d'eIF(iso)4E de tomate (N° d'accession TIGR TC126316) (5'-AAAACAATGGCCACCGAAGCA-3', SEQ ID NO : 7). La RT-PCR est réalisée dans les conditions suivantes : 5 min à 94°C puis 30 cycles (30 sec à 94°C, 30 sec à 53°C, 1 min à 72°C) et 5 min à 72°C.

Le système de vecteur pGEM-T Easy (Promega, Madison, USA) a été utilisé pour cloner l'ADNc après l'amplification.

Au moins 3 clones positifs indépendants ont été séquencés, à partir des deux extrémités, par Genome express (Grenoble, France). Le programme PileUp de GCG (Genetics Computer Group, Madison, USA) a été utilisé pour l'analyse des séquences nucléotidique et peptidique.

La séquence d'ADNc de eIF(iso)4E de piment ainsi obtenue, a une longueur de 660 nucléotides (SEQ ID NO : 3) et une seule phase ouverte de lecture de 609 nucléotides codant pour 202 acides aminés (SEQ ID NO : 4).

Les pourcentages d'identité de séquence les plus importants, déterminés en utilisant le logiciel BLAST sur une fenêtre de comparaison comprenant la totalité de l'ADNc de eIF(iso)4E de piment, ont été obtenus pour l'ADNc de eIF(iso)4E de laitue (AF530163, E=1.10⁻⁵⁹), de pois (AY423377, E=2.10⁻²⁴), de maïs (AF076955, E=4.10⁻¹⁰), d'A. *thaliana* (Y10547, E=6.10⁻⁹) et de blé (M95818, E=9.10⁻⁸).

Les pourcentages d'identité de séquence les plus importants, déterminés en utilisant le logiciel BLAST sur une fenêtre de comparaison comprenant la totalité de la séquence peptidique prédite de eIF(iso)4E de piment, ont été obtenus pour la protéine eIF(iso)4E de laitue (AAP86603, E=8.10⁻⁷⁷) et de blé (AAA34296, E=91.10⁻⁸⁷³), confirmant que l'ADNc de Yolo Wonder obtenu correspond bien à l'ADNc de eIF(iso)4E.

Les régions codantes des gènes *eIF4E* de piment (GenBank n° d'accession : AY122052, RUFFEL et *al.,* 2002) et de *eIF(iso)4E* de piment présentent 57,2% d'identité de séquence (et 48,3% d'identité de séquence au niveau des séquences peptidiques correspondantes).

### EXEMPLE 2 : COMPARAISON DES SEQUENCES DES GENOTYPES pvr6⁺ ET pvr6 CHEZ LE PIMENT : MISE EN EVIDENCE DE MUTATIONS DANS eIF(ISO)4E

Pour déterminer si les variations de séquence peuvent être associées avec l'allèle *pvr6⁺* ou *pvr6,* les séquences nucléotidiques et d'acides aminés correspondantes de l'ADNc *eIF(iso)4E* pleine longueur de 5 lignées de piment différentes ont été alignées :
- La variété Yolo Wonder (YW) est sensible aux potyvirus ;
- La variété Yolo Y (YY) possède l'allèle de résistance *pvr2¹* (résistance au PVY pathotype 0), et est sensible au PVMV ;
- La variété Florida VR2 (F) possède l'allèle de résistance *pvr2²* (résistance aux PVY (0) et (1) et au TEV), et est sensible au PVMV ;
- La variété Perennial (P) possède l'allèle *pvr2³* de résistance partielle au PVY et au potyvirus E et l'allèle *pvr6,* et est sensible au PVMV ;
- La lignée haploïde doublé DH801 issue de la F1 [Perennial x Florida VR2] possède les allèles *pvr2²* et *pvr6,* et est résistante au PVMV.

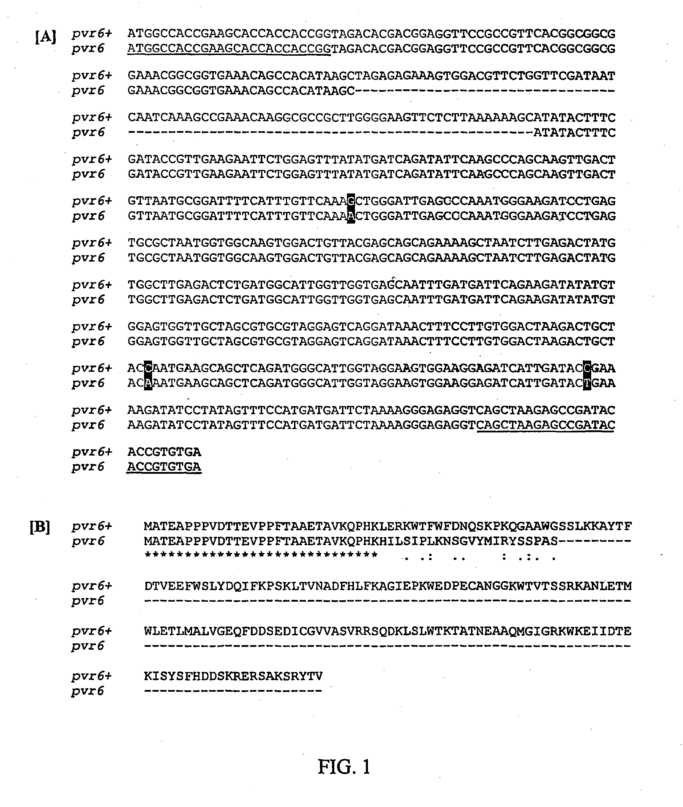

Les résultats de l'alignement des phases ouvertes de lecture de *eIF(iso)4E,* et des séquences d'acides aminés correspondantes, des 5 lignées de piment décrites ci-dessus, sont représentés sur la Figure 1.

Légende de la Figure 1 :
(A) = alignement des séquences nucléotidiques des génotypes *pvr6⁺* et *pvr6* (partie codante)
(B) = alignement des séquences d'acides aminés correspondantes des génotypes *pvr6⁺* et *pvr6*

Les alignements nucléotidiques montrent que la partie codante des allèles *pvr6⁺* des génotypes YW, YY et F sont identiques entre eux à 100%. Les alignements nucléotidiques montrent que la partie codante des allèles *pvr6* des génotypes P et DH801 sont également identiques entre elles à 100%, mais diffèrent du premier groupe par la délétion des nucléotides 89 à 170, par une substitution d'un G par un A à la position 268, d'un C par un A à la position 483, et d'un C par un T à la position 537 (Figure 1A). La délétion de 82 nucléotides modifie la phase ouverte de lecture et introduit un codon stop après l'acide aminé 51. L'alignement des séquences protéiques eIF(iso)4E des génotypes *pvr6⁺* et *pvr6* montre que seuls les 29 premiers acides aminés (sur 202 que compte la protéine) sont conservés (Figure 1B).

### EXEMPLE 3 : SELECTION DE PLANTES POSSEDANT A LA FOIS UN ALLELE MUTE AU LOCUS pvr2 (SEQUENCES eIF4E CORRESPONDANT AUX ALLELES pvr2¹ OU pvr2²) ET L'ALLELE MUTE pvr6 (SEQUENCE eIF(ISO)4E MUTEE) A L'ETAT HOMOZYGOTE

A partir des plantes de piment possédant les allèles *pvr2¹* (SEQ ID NO : 8 de la Demande PCT WO 03/066900) ou *pvr2²* (SEQ ID NO : 22 de la Demande PCT WO 03/066900) à l'état homozygote (les méthodes de sélection de ces mutants sont décrites dans la Demande PCT WO 03/066900), les plantes possédant en outre l'allèle *pvr6* correspondant à *eIF(iso)4E* muté, et donc résistantes au PVMV, sont sélectionnées comme suit :

### Sélection par RT PCR

L'extraction de l'ARN des plantes suit les protocoles d'extraction standard basé sur le protocole fourni par Sigma-Aldrich (St. Louis, USA) avec le produit TRI-Reagent commercialisé par cette même société.

La reverse transcription (RT) de l'ARN en ADNc est réalisé comme suit : un mélange de 7.5 µl d'H₂O, 1 µl d'amorce oligoDT (100 ng/µl), 2.5 µl de dNTP (4mM chacun) et 2 µl d'ARN est incubé 5 min à 65°C, puis 2 min dans la glace. 4 µl de tampon RT Invitrogen (5X) et 2 µl de DTT (0.1M) sont ajoutés, puis le mélange est incubé 2 min à 42°C. 1µl de SuperScript RT II (Invitrogen, 200 U/pl) est ajouté, puis le mélange est incubé 50 min à 42°C. La réaction est inactivée 15 min à 70°C, puis la conservation de l'ADNc produit se fait à 4°C.

La PCR est réalisée en mélangeant 17 µl d'H₂O, 2.5 µl du Tampon PCR Buffer Hifi (10X) (GIBCO/BRL Life Technologies), 1 µl de MgSO4 (50 mM), 1 µl de dNTP (4 mM chacun), 0.5 µl de l'amorce sens 5'-ATGGCCACCGAAGCACCACCACCGG-3' (SEQ ID NO : 8) (10 µm), 0.5 µl de l'amorce anti-sens 5'-TCACACGGTGTATCGGCTCTTAGCT-3' (SEQ ID NO : 9) (10µM) (l'emplacement de ces amorces est indiqué dans la Figure 1A), 0.5 µl de High Fidelity Platinium Taq Polymerase (5U/µl) (GIBCO/BRL Life Technologies) et 2 µl de produit de RT. La PCR est réalisée dans les conditions suivantes : 5 min à 94°C puis 30 cycles (30 sec à 94°C, 30 sec à 64°C, 1 min à 72°C) et 5 min à 72°C.

La migration des produits PCR est réalisée sur gel d'agarose 1% avec le tampon TAE 1X et les fragments amplifiés sont révélés par coloration au Bromure d'éthidium (BET).

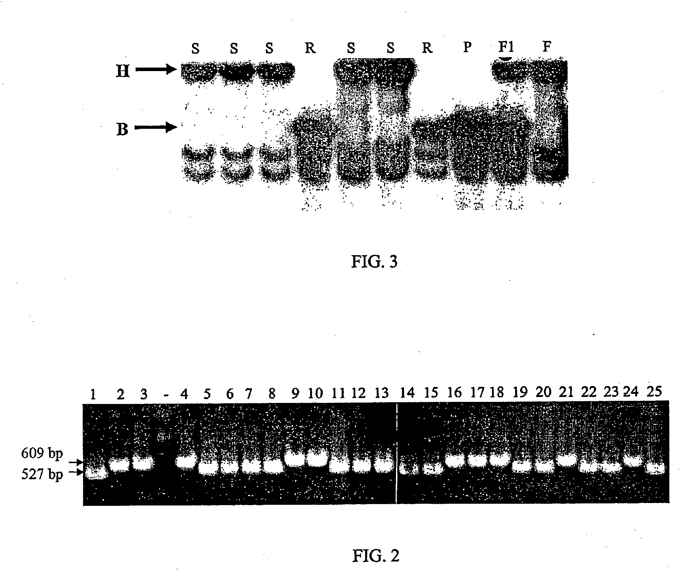

Les résultats sont présentés dans la Figure 2.

Légende de la Figure 2 :

La piste 1 correspond à la RT-PCR mise en oeuvre sur l'ARN du cultivar Perennial (allèle *pvr6*), 2 et 3 aux cultivars Florida VR2 et Yolo Wonder (allèle *pvr6+*). Les pistes 5, 6, 7, 8, 11, 12, 13, 14, 15, 19, 20, 22, 23 et 25 correspondent à la RT-PCR mise en oeuvre dans des descendances résistantes au PVMV et les pistes 4, 9, 10, 16, 17, 18, 21 et 24 à la RT-PCR mise en oeuvre dans des descendances sensibles au PVMV.

Les résultats montrent que les plantes porteuses de l'allèle *eIF(iso)4E* muté (*pvr6*) présentent un produit d'amplification de 527 pb (correspondant à la délétion de 82 pb) alors que les plantes porteuses de l'allèle *eIF(iso)4E* sauvage (*pvr6+*) présentent un produit d'amplification de 609 pb.

### Sélection par RFLP

### 1) Digestion par EcoRV et séparation des fragments de restriction

L'ADN est extrait des plantes selon le protocole de micro extraction décrit par FULTON et al. (Plant Mol. Biol. Reptr. 13(3) : 207-209, 1995).

Il est ensuite digéré par 2,5 U d'enzyme *EcoRV*/µg d'ADN, à 37°C pendant une nuit. Le produit de digestion est déposé sur gel NEB 1X, 1% d'agarose contenant 10µl de BET, pour séparer les fragments de restriction. La migration est effectuée à 25V pendant 24h dans du tampon NEB 1X. après migration, les fragments sont transférés sur membrane de nylon. Les membranes sont rincées dans un bain de SSC 2X durant 10 à 15 min puis séchées à l'air libre et cuites 2h à 80°C.

### 2) Préparation des sondes

L'ADNc *eIF(iso)4E (pvr6⁺)* du génotype Yolo Wonder, obtenu comme décrit dans l'exemple 1, est utilisé comme sonde.

La préparation des sondes marquées au ³²P est effectuée par PCR dans les conditions suivantes :

| | Concentration finale |
|---|---|
| H₂O | 25,6 µl |
| Tp Promega 10 X | 4 µl 1X |
| MgCl₂ Promega | 2,4 µl |
| Mix (ATG 50 µM+dCTP 5 µM)* | 2 µl ATG 2,5 µM ; dCTP 0,25 µM |
| Taq 2U/µl | 1 µl |
| Primer (5pM) | 1 µl |
| α32P-dCTP (1000Ci/mmole, 10µCi/µl) | 3 µl |
| ADN matrice | 1 µl |
| Volume réactionnel final | 40 µl |

| | |
|---|---|
| * : Mix (ATG 50 µM+dCTP 5 µM) pour marquage des sondes RFLP par PCR. | |

Dilution de dATP, dTTP, dGTP à 10 mM, à partir des solutions mères à 100 mM :
- 5 µl dNTP à 100 mM
- 45µl H₂O

Dilution de dCTP à 1 mM, à partir de la solution mère à 100 mM :
- 0,5 µl dCTP à 100 mM
- 49,5 µl H₂O

Mix ATG + dCTP :
- 2,5 µl dATP à 10 mM concentration finale : 50 µM
- 2,5 µl dTTP à 10 mM concentration finale : 50 µM
- 2,5 µl dGTP à 10 mM concentration finale : 50 µM
- 2,5 µl dCTP à 1 mM concentration finale : 5 µM
- 490 µl H₂O

Le marquage des sondes se fait au cours de 30 cycles PCR : 30 sec à 94°C, 45 sec à 52 °C, 1 min 30 à 72 °C.

Une fois marquées, les sondes sont dénaturées, puis hybridées avec les membranes obtenues comme décrit en 1). L'hybridation est effectuée à 65°C pendant au moins 16 heures dans le tampon suivant : pour 500 mL : 21,91 g NaCl; 18,38 g Na Citrate; 380 mL H2O 15 mL SDS 20%; 25 mL NaPO4 1M pH 7,5; 25 mL Denhardt 100X; 5 mL EDTA 0,25M; 50 mL Dextran sulfate 50%).

Les membranes sont ensuite lavées dans du tampon 1% SDS (Serva) 40mM NaPi, préchauffé à 65°C.

Les conditions de lavage sont les suivantes :
- 1 lavage de 20 min. à 65°C sous agitation.
- 1 rinçage de 2-3 min. dans un tampon neuf chauffé à 65°C.

Les résultats de RFLP sont présentés sur la Figure 3.

Légende de la Figure 3 :

La figure montre la différence de profil RFLP observée pour le marqueur eIF(iso)4E entre les génotypes Perennial avec l'allèle *pvr6* (ligne P), Florida VR2 avec l'allèle *pvr6+* (ligne F), l'hybride F1 Perennial X Florida VR2 (ligne F1) et des descendances résistantes (lignes R) ou sensibles (lignes S) au PVMV.

Les résultats montrent que les plantes sensibles avec l'allèle *pvr6+* possèdent le fragment de restriction H alors que les plantes résistantes avec l'allèle *pvr6* (en plus de l'allèle *pvr2²)* possèdent le fragment de restriction B.

### EXEMPLE 4 : TEST DE RESISTANCE AU PVMV ET EXPRESSION TRANSITOIRE DE eIF(iso)4E (pvr6⁺) OU eIF4E (pvr2⁺) DANS UN GENOTYPE DE PIMENT RESISTANT AU PVMV

### Test de la résistance du piment au PVMV

Ce test est utilisé pour vérifier que les plantes porteuses des allèles de résistance *pvr2¹* (ou *pvr2²*) et *pvr6* sont bien résistantes au PVMV.

Le matériel viral employé dans les tests d'inoculation correspond à l'isolat PVMV-IC isolé en Cote d'Ivoire et fourni par J.C. THOUVENEL (IRD, Montpellier, France) et aux isolats décrits dans la publication de CARANTA *et al.* (1996, précité). Les isolats sont maintenus selon la procédure BOS (BOS, Meded. Fac. Landbouwwet Gent. 34 : 875-887, 1969) et multipliés sur des plants de *Capsicum annuum cv.* Yolo Wonder avant inoculation mécanique des plantes de piment au stade cotylédons ou 2 feuilles étalées.

L'inoculum viral est préparé comme décrit dans CARANTA et *al.* (1996, précité). Les tests d'inoculation sont réalisés en conditions contrôlées dans des chambres climatisées « insect-proof ». 4 semaines après inoculation, toutes les plantes sont évaluées individuellement pour la présence ou l'absence de l'antigène de la protéine capside du PVMV par test DAS-ELISA comme décrit par CARANTA et *al.* (1996, précité). D'autres protocoles parfaitement connus de l'homme du métier peuvent également être utilisés pour l'inoculation mécanique et la détection de PVMV dans les plantes de piment.

Ce test de résistance permet de suivre la coségrégation totale entre la résistance au PVMV et la présence des allèles *pvr2¹* (ou *pvr2²*) correspondant à des formes mutées de *eIF4E* et la présence de l'allèle *pvr6* correspondant à la forme mutée de *eIF(iso)4E.*

### Expression transitoire des ADNc eIF(iso)4E ou eIF4E de Yolo Wonder dans un génotype de piment résistant au PVMV (combinant pvr2² + pvr6) pour validation du rôle indépendant des protéines eIF(iso)4E et eIF4E dans la sensibilité au PVMV

Afin de valider l'hypothèse que le PVMV est capable d'utiliser de façon indépendante soit l'allèle *pvr2⁺* (correspondant à l'allèle sauvage *eIF4E*) soit l'allèle *pvr6⁺* (correspondant à l'allèle sauvage *eIF(iso)4E)* pour accomplir son cycle infectieux, des expériences d'expression transitoire des ADNc eIF4E ou eIF(iso)4E de Yolo Wonder *via* un vecteur viral PVX (*Potato virus X*) (CHAPMAN et al., Plant J. 2(4) : 549-557, 1992 ; BAULCOMBE et al., Plant J. 7 : 1045-1053, 1995) ont été réalisée sur le génotype résistant DH801, porteur des allèles *pvr2²* et *pvr6.*

Les ORFs de *eIF4E* des génotypes Yolo Wonder (*pvr2+*) et Florida VR2 *(pvr2²)* ont été clonés dans le vecteur d'expression pPVX201 (BAULCOMBE *et al.,* 1995, précité) au site de clonage *Cla*I et *Sal*I, pour obtenir les vecteurs pPVXeYW et pPVXeF, respectivement. De la même façon, les ORF de *eIF(iso)4E* des génotypes Yolo Wonder (*pvr6+*) et Perennial (*pvr6*) ont été cloné dans pPVX201 pour obtenir les vecteurs d'expression pPVX(iso)eYW et pPVX(iso)eP, respectivement. Ces plasmides sont inoculés mécaniquement sur *Nicotiana benthamiana.* Dix jours après inoculation, les feuilles inoculées de *N. benthamiana* sont utilisées comme inoculum pour les tests d'expression transitoire chez les génotypes de piment Yolo Wonder et DH801. Dix jours après inoculation des piments avec le vecteur d'expression PVX, les mêmes feuilles sont inoculées avec le PVMV-IC comme décrit ci-dessus.

L'accumulation du vecteur PVX et du PVMV est évaluée par DAS-ELISA et RT-PCR sur les feuilles inoculées, 10 jours après l'inoculation du PVMV. La RT-PCR (conditions classiques connues de l'homme du métier) permettant de détecter l'accumulation du PVX est réalisée avec les amorces suivantes : sens 5'-CCGATCTCAAGCCACTCTCCG-3' (SEQ ID NO : 10) et anti-sens 5'-CCTGAAGCTGTGGCAGGAGTTG-3' (SEQ ID NO : 11). La RT-PCR (conditions classiques connues de l'homme du métier) permettant de détecter l'accumulation du PVMV est réalisée avec les amorces dégénérées suivantes : sens (définie dans le gène Nib) 5'-GGNAARGCNCCNTAYAT-3' (SEQ ID NO : 12) et anti-sens (définie dans le gène CP) 5'-CGCGCTAATGACATATCGGT-3' (SEQ ID NO : 13).

Le génotype résistant au PVMV DH801 est co-inoculé avec un plasmide recombinant (possédant eIF4E ou eIF(iso)4E) et avec l'isolat PVMV-IC. Des expériences témoins sont réalisées en parallèle en exprimant, chez DH801, soit l'allèle *pvr2²* (du génotype Florida VR2) soit l'allèle *pvr6* (du génotype Perennial) pour vérifier que les formes mutées ne restaurent pas la sensibilité au PVMV.

Les résultats sont résumés dans le tableau ci-dessous indiquant le rapport du nombre de feuilles infectées par le PVMV sur le nombre de feuilles inoculées au PVMV dans les expériences d'expression transitoire.

| Feuilles inoculées avec | Génotypes de piment | |
|---|---|---|
| | Yolo Wonder | DH801 |
| PVMV | 20/20 | 0/20 |
| pPVX201 + PVMV | 20/20 | 0/20 |
| pPVXeYW + PVMV | 20/20 | 13/30 |
| pPVXeF + PVMV | 20/20 | 0/30 |
| pPVX (iso) eYW + PVMV | 20/20 | 5/30 |
| pPVX(iso)eP + PVMV | 20/20 | 0/30 |

Ces expériences montrent qu'à la fois l'expression du gène *eIF4E* et indépendamment celle du gène *eIF(iso)4E* (gènes issus du génotype sensible Yolo Wonder), permettent au PVMV de se multiplier dans le génotype résistant DH801. A partir de ces expériences, nous pouvons conclure que le PVMV est capable d'utiliser soit la protéine sauvage eIF4E soit la protéine sauvage eIF(iso)4E, pour réaliser son cycle infectieux, et que des mutations dans les deux protéines sont nécessaires pour rendre la plante résistante.

### EXEMPLE 5 : OBTENTION DE PLANTES RESISTANTES AU PVMV PAR INACTIVATION DE eIF(iso)4E

L'allèle du gène *eIF(iso)4E* qui confère la résistance au PVMV (*pvr6*), chez des plantes porteuses des allèles de résistance *pvr2¹* (ou *pvr2²*), peut être transféré *in planta* par des méthodes de type mutagenèse dirigée (HOHN et al., Proc. Natl. Acad. Sci. USA. 96 : 8321-8323, 1999), ou recombinaison homologue (KEMPLIN et al., Nature 389 : 802-803, 1997 ; JONES et al., Transgen. Res. 1 : 285-297, 1992 ; BEVAN, Nucleic Acid research. 12 : 8711-8721, 1984).

Des plantes porteuses des allèles de résistance *pvr2¹* (ou *pvr2²*) résistantes au PVMV, peuvent également être obtenues par knock-out du gène *eIF(iso)4E* endogène par des méthodes de type « gene silencing » (Post Transcriptionnal Gene Silencing ou PTGS). Un knock-out spécifique par PTGS peut être réalisé en le dirigeant contre le 5' ou 3' UTR du gène *eIF(iso)4E* endogène. Cette spécificité du knock-out par PTGS contre les 5' ou 3'UTR, est basée sur les nouvelles données issues de la compréhension du mécanisme de PTGS (NISHIKURA, Cell 107 : 415-418, 2001).

## Revendications

1. Procédé de sélection de plantes résistantes au PVMV, **caractérisé en ce qu'**il comprend la recherche, dans les plantes à tester, des formes des protéines eIF4E, et eIF(iso)4E présentes chez lesdites plantes, et la sélection des plantes qui :
a) n'expriment aucune protéine eIF4E (dénommée ci-après : « protéine eIF4E de type sauvage ») comprenant une région définie par la séquence générale (I) suivante :
DX₁X₂X₃X₄KSX₅QX₆AWGSSX₇RX₈X₉YTFSX₁₀VEX₁₁FWX₁₂X₁₃YNNIHX₁₄PSKLX₁₅X₁₆ GAD
dans laquelle :
- X_{1,} X_{2,} X_{3,} X_{4,} X_{6,} X₇, X_{8,} X₉, X₁₀, X_{12,} X₁₃, X_{15,} et X₁₆ représentent chacun un acide aminé neutre ;
- X₅ et X₁₄ représentent un acide aminé basique ;
- X₁₁ représente un acide aminé acide ;
- D, K, S, Q, A, W, G, R, Y, T, F, V, E, N, I, H, P, et
L ont leur signification usuelle en code 1-lettre ; et expriment une protéine eIF4E mutante comprenant une région dérivée de celle définie par la séquence (I) ci-dessus, par substitution d'au moins un acide aminé neutre de ladite séquence (I) par un acide aminé chargé, de préférence un acide aminé basique et/ou substitution d'au moins un acide aminé chargé de ladite séquence (I) par un acide aminé neutre ou un acide aminé de charge opposée ;
et
b) n'expriment aucune protéine eIF(iso)4E fonctionnelle.

2. Procédé selon la revendication 1, **caractérisé en ce qu'**il comprend la sélection des plantes exprimant une protéine eIF(iso)4E non-fonctionnelle délétée d'au moins une portion de la séquence d'une protéine eIF(iso)4E sauvage, ladite délétion comprenant un ou plusieurs des résidus tryptophane du domaine IF4E de ladite protéine correspondant aux positions 34, 37, 50, 67, 96, 107, 121, et 156 de la séquence SEQ ID NO : 4.

3. Procédé selon une quelconque des revendications 1 ou 2, **caractérisé en ce qu'**il comprend la sélection des plantes exprimant une protéine eIF4E mutante comprenant une région dérivée de celle définie par la séquence (I) ci-dessus, par substitution :
- d'au moins un des acides aminés X_{1,} X_{2,} X₃ ou X_{4,} de ladite séquence (I) par un acide aminé chargé, et
- d'au moins un des autres acides aminés neutres de ladite séquence (I) par un acide aminé chargé et/ou
- d'au moins un des acides aminés chargés de ladite séquence (I) par un acide aminé neutre ou un acide aminé de charge opposée.

4. Procédé selon la revendication 3, **caractérisé en ce qu'**il comprend la sélection des plantes exprimant une protéine eIF4E mutante comprenant une région dérivée de celle définie par la séquence (I) ci-dessus, par substitution :
- de l'acide aminé neutre X₃ de la séquence (I) par un acide aminé basique ;
- de l'acide aminé neutre X₇ de la séquence (I) par un acide aminé basique ;
- du résidu aspartate en position C-terminale de la séquence (I) par un acide aminé neutre.

5. Procédé d'obtention d'une plante résistante au PVMV, **caractérisé en ce qu'**il comprend :
- le remplacement des allèles du gène *eIF4E* présents chez ladite plante par des allèles codant pour une protéine eIF4E mutante telle que définie dans une quelconque des revendications 1, 3 ou 4 ;
et
- le remplacement des allèles du gène *eIF(iso) 4E* présents chez ladite plante par des allèles codant pour une protéine eIF(iso)4E mutante non-fonctionnelle , ou l'inactivation, directe ou épigénétique, dudit gène *eIF(iso)4E.*

6. Procédé d'obtention d'une plante résistante au PVMV, **caractérisé en ce qu'**il est mis en oeuvre à partir d'une plante dont tous les allèles du gène *eIF4E* codent pour une protéine eIF4E mutante telle que définie dans une quelconque des revendications 1, 3 ou 4, et **en ce qu'**il comprend le remplacement des allèles du gène *eIF(iso) 4E* présents chez ladite plante par des allèles codant pour une protéine eIF(iso)4E mutante non-fonctionnelle , ou l'inactivation, directe ou épigénétique, dudit gène *eIF(iso) 4E.*

7. Procédé d'obtention d'une plante résistante au PVMV, **caractérisé en ce qu'**il est mis en oeuvre à partir d'une plante dont tous les allèles du gène *eIF(iso)4E* codent pour une protéine eIF(iso)4E mutante non-fonctionnelle , ou à partir d'une plante dans laquelle ledit gène *eIF(iso)4E* a été inactivé, et **en ce qu'**il comprend le remplacement des allèles du gène *eIF4E* présents chez ladite plante par des allèles codant pour une protéine eIF4E mutante telle que définie dans une quelconque des revendications 1, 3 ou 4.

8. Procédé selon une quelconque des revendications 1 à 7, **caractérisé en ce qu'**il est mis en oeuvre sur une plante choisie parmi les solanacées, les cucurbitacées, les crucifères, les fabacées, et les composées.

## Claims

1. Process for the selection of PVMV-resistant plants, **characterised in that** it comprises the search, in the plants to be tested, for forms of the eIF4E and eIF(iso)E proteins present in said plants, and the selection of those plants that:
a) do not express any eIF4E protein (hereinafter referred to as "wild-type eIF4E protein") comprising a region defined by the following general sequence (I):
DX₁X₂X₃X₄K₅XQX₆AWGSSX₇RX₈X₉YTFSX₁₀VEX₁₁FWX₁₂X₁₃YNNIHX₁₄PSKLX₁₅X₁₆GAD
in which:
- X₁, X₂, X₃, X₄, X₆, X₇, X₈, X₉, X₁₀, X₁₂, X₁₃, X₁₅ and X₁₆ each represent a neutral amino acid;
- X₅ and X₁₄ represent a basic amino acid;
- X₁₁ represents an acid amino acid;
- D, K, S, Q, A, W, G, R, Y, T, F, V, E, N, I, H, P and L each have their usual meaning in 1-letter code;
and express a mutant eIF4E protein comprising a region derived from that defined by sequence (I) above, by means of the substitution of at least one neutral amino acid of said sequence (I) by a charged amino acid, preferably a basic amino acid and/or the substitution of at least one charged amino acid of said sequence (I) by a neutral amino acid or an oppositely charged amino acid;
and
b) do not express any functional eIF(iso)4E protein.

2. Process according to claim 1, **characterised in that** it comprises the selection of those plants expressing a non-functional eIF(iso)4E deleted from at least one portion of the sequence of a wild eIF(iso)4E protein, said deletion comprising one or more of the tryptophan residues of the IF4E domain of said protein corresponding to the positions 34, 37, 50, 67, 96, 107, 121 and 156 of the sequence SEQ ID NO: 4.

3. Process according to either one of claims 1 and 2, **characterised in that** it comprises the selection of plants expressing a mutant eIF4E protein comprising a region derived from that defined by sequence (I) above, by means of the substitution:
- of at least one of the amino acids X₁, X₂, X₃ or X₄, of said sequence (I) by a charged amino acid, and
- of at least one of the other neutral amino acids of said sequence (I) by a charged amino acid and/or
- of at least one of the charged amino acids of said sequence (I) by a neutral amino acid or an oppositely charged amino acid.

4. Process according to claim 3, **characterised in that** it comprises the selection of plants expressing a mutant eIF4E protein comprising a region derived from that defined by sequence (I) above, by means of the substitution:
- of the neutral amino acid X₃ of the sequence (I) by a basic amino acid;
- of the neutral amino acid X₇ of the sequence (I) by a basic amino acid;
- of the aspartate residue in C-terminal position of the sequence (I) by a neutral amino acid.

5. Process for obtaining a PVMV-resistant plant, **characterised in that** it comprises:
- the replacement of the alleles of the *eIF4E* gene present in said plant by alleles coding for a mutant eIF4E protein such as defined in any one of claims 1, 3 or 4;
and
- the replacement of the alleles of the *eIF(iso)4E* gene present in said plant by alleles coding for a non-functional mutant eIF(iso)4E protein, or the direct or epigenetic inactivation of said *eIF(iso)4E* gene.

6. Process for obtaining a PVMV-resistant plant, **characterised in that** it is implemented based on a plant all of whose alleles of the *eIF4E* gene code for a mutant eIF4E protein as defined in any one of claims 1, 3 or 4, and **in that** it comprises the replacement of the alleles of the *eIF(iso)4E* gene present in said plant by alleles coding for a non-functional mutant eIF(iso)4E protein, or the direct or epigenetic inactivation of said *eIF(iso)4E* gene.

7. Process for obtaining a PVMV-resistant plant, **characterised in that** it is implemented based on a plant all of whose alleles of the *eIF(iso)4E* gene code for a mutant eIF(iso)4E protein, or based on a plant in which said *eIF(iso)4E* gene has been inactivated, and **in that** it comprises the replacement of the alleles of the *eIF4E* gene present in said plant with alleles coding for a mutant eIF4E protein such as defined in any one of claims 1, 3 or 4.

8. Process according to any one of claims 1 to 7, **characterised in that** it is implemented on a plant chosen from among the Solanaceae, Cucurbitacae, Brassicacae, Fabaceae, and Compositae.

## Patentansprüche

1. Verfahren zur Auswahl von gegen PVMV resistenten Pflanzen, **dadurch gekennzeichnet, dass** es eine Suche in den zu untersuchenden Pflanzen nach Formen der eIF4E- und eIF(iso)4E-Proteine umfasst, die in den Pflanzen vorliegen, und die Auswahl der Pflanzen, die
a) kein eIF4E-Protein exprimieren (nachfolgend als "Wildtyp eIF4E-Protein" bezeichnet), das die durch die allgemeine folgende Sequenz (I) definierte Region umfasst:
DX₁X₂X₃X₄KSX₅QX₆AWGSSX₇RX₈X₉YTFSX₁₀VEX₁₁FWX₁₂X₁₃YNNIHX₁₄P SKLX₁₅X₁₆GAD, in welcher
- X₁, X₂, X₃, X₄, X₆, X₇, X₈, X₉, X₁₀, X₁₂, X₁₃, X₁₅ und X₁₆ jeweils eine neutrale Aminosäure wiedergeben;
- X₅ und X₁₄ eine basische Aminosäure wiedergeben;
- X₁₁ eine saure Aminosäure wiedergibt;
- D, K, S, Q, A, W, G, R, Y, T, F, V, E, N, I, H, P und L ihre übliche Bedeutung nach dem Ein-Buchstabencode aufweisen;
und ein mutiertes eIF4E-Protein exprimieren, das eine Region umfasst, die von der durch die oben definierte Sequenz (I) abgeleitet ist, durch Substitution wenigstens einer neutralen Aminosäure der Sequenz (I) durch eine geladene Aminosäure, bevorzugt eine basische Aminosäure und/oder Substitution wenigstens einer geladenen Aminosäure der Sequenz (I) durch eine neutrale Aminosäure oder eine entgegengesetzt geladene Aminosäure;
und
b) kein funktionelles eIF(iso)4E-Protein exprimieren.

2. Verfahren nach Anspruch 1, **dadurch gekennzeichnet, dass** es die Auswahl von Pflanzen umfasst, die ein nicht funktionelles eIF(iso)4E-Protein exprimieren, das aus wenigstens einem Teil der Sequenz eines Wildtyp- eIF(iso)4E-Proteins deletiert ist, wobei die Deletion einen oder mehrere Tryptophanreste der IF4E-Domäne des Proteins umfasst, die den Positionen 34, 37, 50, 67, 96, 107, 121 und 156 der SEQ ID Nr. 4 entsprechen.

3. Verfahren nach einem der Ansprüche 1 oder 2, **dadurch gekennzeichnet, dass** es die Auswahl von Pflanzen umfasst, die ein mutiertes eIF4E-Protein exprimieren, das eine Region umfasst, die von der durch die oben definierte Sequenz (I) abgeleitet ist, durch Substitution:
- wenigstens einer der Aminosäuren X₁, X₂, X₃ oder X₄ der Sequenz (I) durch eine geladene Aminosäure, und
- wenigstens einer der anderen neutralen Aminosäuren der Sequenz (I) durch eine geladene Aminosäure, und/oder
- wenigstens einer geladenen Aminosäure der Sequenz (I) durch eine neutrale Aminosäure oder eine entgegengesetzt geladene Aminosäure.

4. Verfahren nach Anspruch 3, **dadurch gekennzeichnet, dass** es die Auswahl der Pflanzen umfasst, die eine mutiertes eIF4E-Protein exprimieren, das eine Region umfasst, die von der durch die oben definierte Sequenz (I) abgeleitet ist, durch Substitution:
- der neutralen Aminosäure X₃ der Sequenz (I) durch eine basische Aminosäure;
- der neutralen Aminosäure X₇ der Sequenz (I) durch eine basische Aminosäure;
- eines Aspartatrests in der C-terminalen Position der Sequenz (I) durch eine neutrale Aminosäure.

5. Verfahren zum Erhalt einer gegen PVMV resistenten Pflanze, **dadurch gekennzeichnet, dass** es umfasst:
- Ersetzen der in der Pflanze vorliegenden Allele des *eIF4E*-Gens durch Allele, die für ein mutiertes eIF4E-Protein codieren, wie es in einem der Ansprüche 1, 3 oder 4 definiert ist;
und
- Ersetzen der in der Pflanze vorliegenden Allele des *eIF(iso)4E-Gens* durch Allele, die für ein mutiertes nicht funktionelles eIF(iso)4E-Protein codieren, oder direkte oder epigenetische Inaktivierung des *eIF(iso)4E*-Gens.

6. Verfahren zum Erhalt einer gegen PVMV resistenten Pflanze, **dadurch gekennzeichnet, dass** es ausgehend von einer Pflanze eingesetzt wird, in der alle Allele des *eIF4E*-Gens für ein mutiertes eIF4E-Gen codieren, wie es in einem der Ansprüche 1, 3 oder 4 definiert ist, und dass es das Ersetzen der in der Pflanze vorliegenden Allele der *eIF(iso)4E-Gene* durch Allele umfasst, die für ein mutiertes nicht funktionelles eIF(iso)4E-Protein codieren, oder die direkte oder epigenetische Inaktivierung des *eIF(iso)4E-Gens.*

7. Verfahren zum Erhalt einer gegen PVMV resistenten Pflanze, **dadurch gekennzeichnet, dass** es ausgehend von einer Pflanze eingesetzt wird, in der alle Allele des *eIF(iso)4E*-Gens für ein mutiertes nicht funktionelles *eIF(iso)4E*-Gen codieren oder ausgehend von einer Pflanze, in der das *eIF(iso)4E*-Gen inaktiviert wurde, und dass es das Ersetzen der in der Pflanze vorliegenden Allele des eIF4E-Gens durch Allele umfasst, die für ein mutiertes eIF4E-Protein codieren, wie es in einem der Ansprüche 1, 3 oder 4 definiert ist.

8. Verfahren nach einem der Ansprüche 1 bis 7, **dadurch gekennzeichnet, dass** es ausgehend von einer Pflanze eingesetzt wird, die ausgewählt ist aus Solanaceen, Cucurbitaceen, Cruciferen, Fabaceen und Korbblütlern.
